(19) Europäisches Patentamt
European Patent Office
Office européen des brevets

(11) Publication number: **0 040 099**
**A1**

(12) **EUROPEAN PATENT APPLICATION**

(21) Application number: 81302110.2

(22) Date of filing: 12.05.81

(51) Int. Cl.$^3$: C 07 H 21/00

(30) Priority: 14.05.80 US 149685

(43) Date of publication of application: 18.11.81 Bulletin 81/46

(84) Designated Contracting States: AT BE CH DE FR GB IT LI LU NL SE

(71) Applicant: ENS BIOLOGICALS INC.
Suite 507 20, Victoria Street
Toronto Ontario, M5C 2N8(CA)

(72) Inventor: Bender, Robert
c/o Suite 507 20 Victoria Street
Toronto, Ontario M5C 2N8(CA)

(72) Inventor: Ogilvie, Kelvin K.
54, place de Bretagne
Candiac Quebec J5R 3M8(CA)

(74) Representative: Eyles, Christopher Thomas et al,
BATCHELLOR, KIRK & EYLES 2 Pear Tree Court
Farringdon Road
London, EC1R 0DS(GB)

(54) **Polynucleotide synthesis.**

(57) Polynucleotides are produced in a semi-automated process using a solid, polymeric support to which an initial nucleotide unit is initially chemically attached. In subsequent chain extension steps, the polymer-nucleoside is reacted with a predetermined 5'-blocked nucleoside complex and blocked phosphodichloridite (which may be prereacted), following suitable unblocking of the 5'-position of the polymer-nucleotide complex. A polynucleotide of any desired chain length and chain sequence is built up sequentially in this way. Oxidation of all phosphite linking groups to phosphate is conducted in a single step after chain extension has been completed.

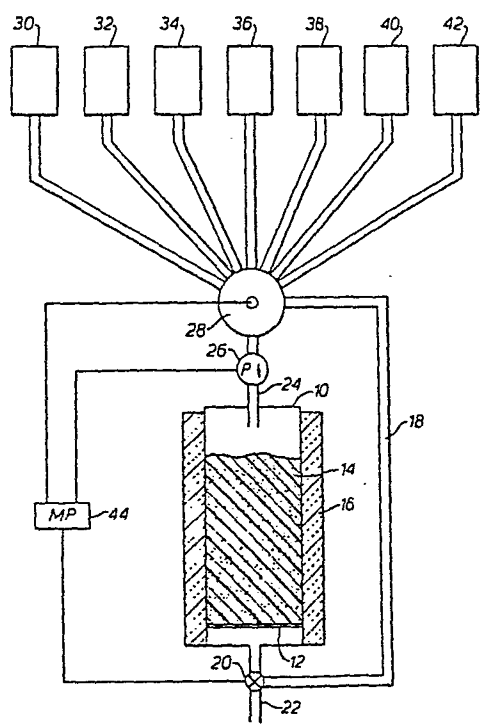


EP 0 040 099 A1

This invention relates to polynucleotide synthesis, and nucleoside complexes useful therein.

Nucleic acids and polynucleotides are of fundamental biochemical and biological importance, in essential life processes. Deoxyribonucleic acid (DNA), the chemical basis of genes, is itself a polynucleotide, having the basic structural unit:

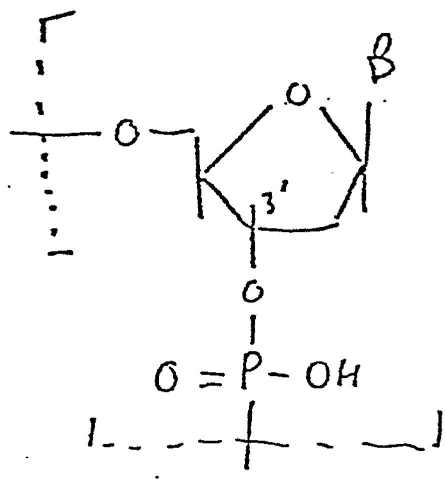

(1)

wherein B represents a purine or pyrimidine base group independently selected from thymine, guanine, cytosine and adenine. The sequence of B-groups along the chain is distinctive of various genes and codes genetic information. DNA segments can be synthesized by stepwise reaction procedures including initially the coupling together of a nucleoside II and a nucleoside III, through a suitable phosphorus containing coupling reagent, for example phosphodichloridite, to produce a dinucleotide IV, and then similar stepwise chain extension steps to form polynucleotides.

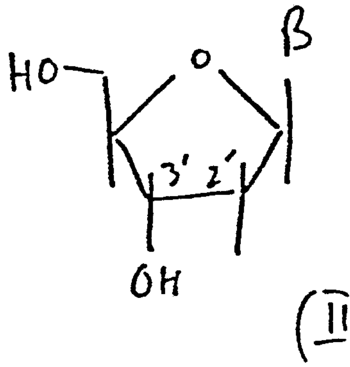

(II)

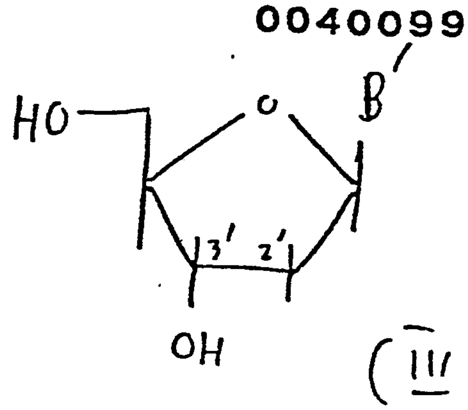

(III)

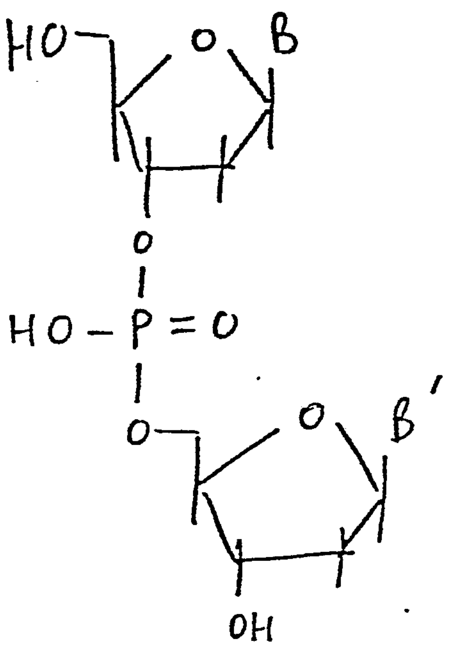

(IV)

Suitable chemical protection must be afforded to reactive groups which are not to participate in the reaction at any given stage. Thus, for the first step of the above reaction the 5'-hydroxyl on nucleoside II and the hydroxyl group of phosphodichloridite must be blocked with unreactive groups, thus:

0040099

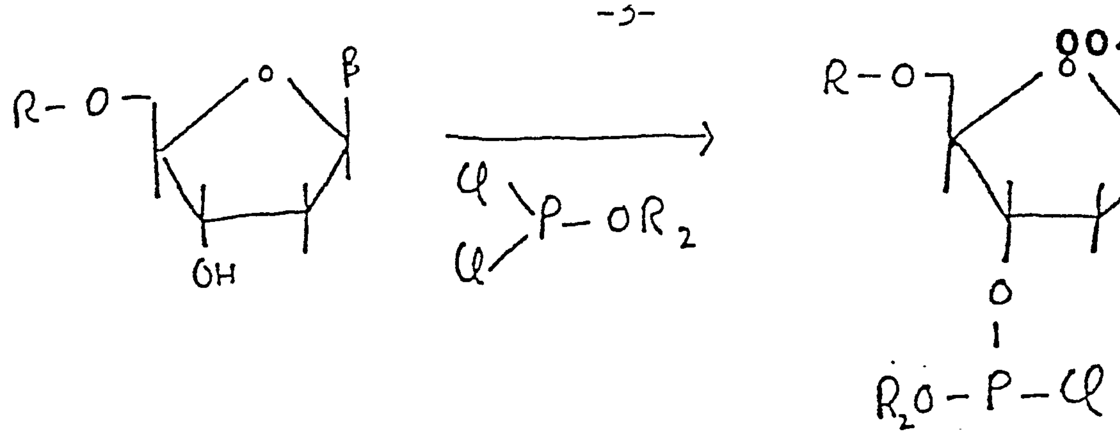

(V)

wherein R and R" represent relatively unreactive blocking groups. Then, 5'-blocked nucleotide V is reacted with 3'-blocked nucleoside III to ensure 3'-5' linkage, thus

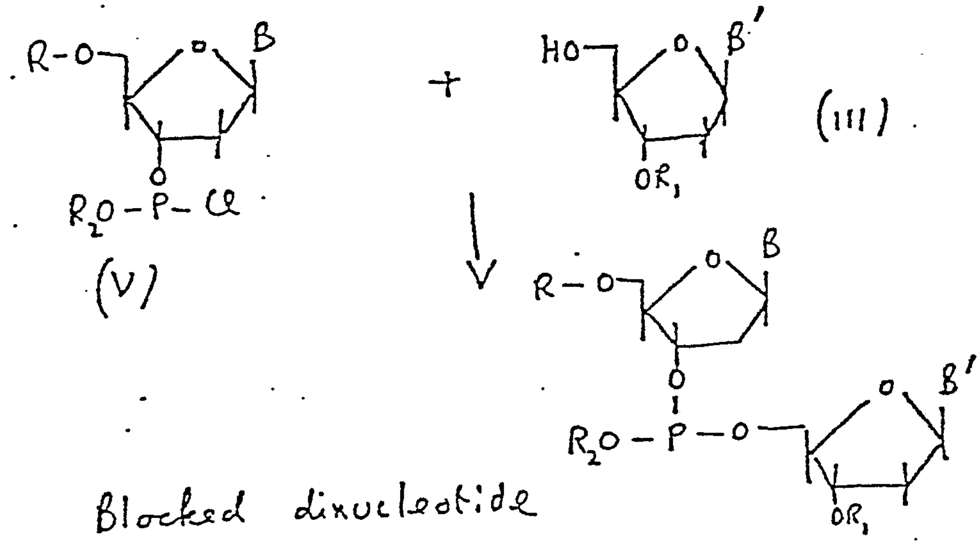

(V) (III) (IV),

Blocked dinucleotide

There must then follow reaction steps of removal of blocking groups, oxidation of the phosphite linkage groups to phosphate, and the repeat of the steps to add more units to the chain. Purine and pyrimidine units B may also need amino protection during the synthesis, in the known way.

In the case of ribonucleic acid (RNA), the basic nucleoside unit is:

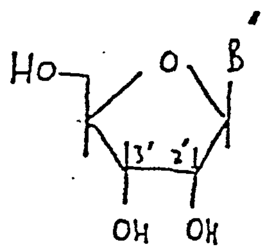

(VI)

wherein B" represents a purine-pyrimidine base selected from

uracil, guanine, adenine and cytosine. In both messenger RNA and transfer RNA, the nucleoside units form 3'-5' linkages through phosphate groups. In synthesis of RNA, there is an extra - OH group to be blocked, in order to direct the coupling reactions in a desired, single mode.

The resulting products are synthetic replicas of natural DNA or RNA segments, and by proper arrangement of the sequence of the base units thereon, specific genes can be synthesized. This synthetic genetic material can be suitably introduced into viable biological cells, e.g. bacterial cells, to modify or enhance the cell's production of commercially useful proteins (human growth hormone, insulin, somatostation and others).

The synthesis of polynucleotides can be a very tedious process, in which a substantial number of chemical reaction steps must be conducted sequentially. When each reaction step is conducted in solution, there is obtained, at the end of each step, a reaction solution containing a mixture of desired product, byproduct, unreacted starting material etc. After each step, product extraction and separation procedures must be adopted. Otherwise an impossibly complicated product mixture is formed for later separation. The increased possibilities for subsequent side reactions with a complicated product mixture decrease the final yield of desired polynucleotide product. Each synthetic step must be chosen and conducted carefully so as to avoid adverse effects on intermediate products.

It is an object of the present invention to provide a

novel method of synthesizing polynucleotides.

It is a further object to provide a polynucleotide synthesis process which results in improved yields of desired polynucleotides, with reduced problems of separation of desired from undesired material.

The present invention provides a method for synthesizing polynucleotides, involving "solid phase" reaction, in which a nucleoside is reacted with a suitably derivatized solid polymer, to form a solid polymer-nucleoside complex. Chain extension is conducted by reacting the solid polymer-nucleoside complex with another appropriately blocked nucleoside, either pre-reacted with a suitable coupling reagent such as phosphodichloridite or in a two step process of first reacting with a blocked coupling reagent and then reacting the intermediate complex, after appropriate de-protection, with the nucleoside, to form a second, chain-extended solid polymer-nucleotide-nucleoside complex. The reactions can be repeated, to build up longer chains of nucleotide units attached to the polymer, as a solid material. As a final or close to final step, the nucleotide chain is split from the polymer, to provide a polynucleotide.

Thus according to one aspect of the present invention a process for preparing polynucleotides of predetermined chain length and nucleotide unit sequence, comprises:

(i) reacting a solid polymer which has side groups of formula -X-CO-Y in which Y represents hydroxyl, chloro, bromo or iodo and X represents a chemical group having at least 6 linearly arranged atoms therein, with a pre-selected partially blocked nucleoside of general formula

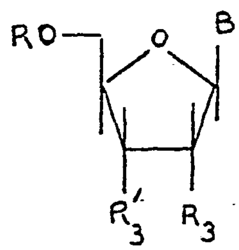

(A)

in which B represents a base selected from uracil thymine, guanine, adenine and cytosine, R represents a blocking group, and either $R_3'$ is hydroxyl and $R_3$ is hydrogen or blocked hydroxyl or $R_3'$ is blocked hydroxyl and $R_3$ is hydroxyl to form a polymer-nucleoside product coupled through an ester linkage;

(ii) reacting the polymer-nuceloside coupled product so formed to replace group RO at position 5' with a hydroxyl group;

(iii) reacting the 5' -unblocked polymer-nucleoside coupled product of step (ii) with a blocked phosphodichloridite and a

preselected, blocked nucleoside of general formula (A) given above, which blocked phosphodichloridite and nucleoside may have been prereacted;

(iv) repeating steps (ii) and (iii) above sequentially, a predetermined number of times, with preselected, blocked nucleosides of general formula (A) given above to yield a polymer-linked polynucleotide chain of predetermined unit sequence;

(v) oxidizing the polymer-polynucleotide product of step (iv) to convert phosphite groups therein to phosphate groups;

(vi) removing the residual blocking groups and reacting the product to cleave the ester linkage of the polynucleotide to the polymer, so as to recover a polynucleotide of predetermined unit sequence and chain length.

The term "polynucleotide" used here is intended to mean linear chains of nucleotides, based on deoxyribose units or ribose units, of at least three units in length. Sometimes the term "oligonucleotides" is used to denote nucleotide chains having from three to about thirty units in length.

The preparation of polynucleotides according to this invention has significant advantages over conventional solution synthesis methods. It simplifies and reduces the number of steps necessary to produce a polynucleotide with a given number of units. The required number of blocking and unblocking reactions is reduced. Blocked nucleoside or nucleotide compounds can be prepared and stored as bench reagents. The process lends itself well to semi-automatic operation. Unwanted side products can be

significantly reduced by preventing the growth of nucleotide chains other than those of predetermined sequence. Desired products are readily separated from unwanted byproducts, by simple phase separation methods.

In another aspect, the invention also provides a novel blocking method for a polynucleotide, which comprises blocking the hydroxyl group of the phosphodichloridite coupling reagent with a methyl group during reaction of the phosphodichloridite with the nucleoside and during subsequent chain extension thereof, and subsequently removing the methyl blocking group with tertiarybutylamine or ammonium hydroxide. The treatment with t-butylamine is very mild and avoids substantial risk of undesired side reactions elsewhere on the chain structure. t-Butylamine is non-toxic so that its residue does not give rise to concern if left in the product. The use of ammonium hydroxide is preferred in some respects, in that it will in addition cleave the nucleotide chain from the polymer, and hence may eliminate a process step.

The derivatized solid polymer used in the invention is one having a plurality of chemical side groupings protruding from the polymer backbone chain, of formula Pol-X-COY, where Pol represents the polymer backbone, Y represents hydroxyl or halogen, and X represents a chain having at least six linearly arranged atoms therein and comprising carbon-carbon chains optionally interrupted by one or more linkages selected from ester linkages amide linkages and ether linkages. The group COY, i.e. the acid or acid haloyl group, is consequently separated from

the substrate polymer backbone by at least 6 atoms. By use of such derivatized polymers, it has been found that the yields of reaction of derivatized polymer and polynucleoside in the first coupling step of the synthesis are very significantly increased. This may be due to substantial lack of steric hindrance, as compared with reactions involving polymers having shorter side chains giving less separation of reactive functional groups from the polymer backbone. It is imperative that high yields of first intermediate product be obtained, if reasonable yields of final product are eventually to be obtained.

The polymers useful as substrates are substantially insoluble in the chosen reaction medium, should have a degree of rigidity, surface impermeability, and a stability sufficient to withstand subsequent chain extension etc. reactions of nucleotides. They are used in the form of solid powders or granules. A very wide choice of different polymers is available for this use. They should have reactive surface side groups which can be chemically modified to derivatize the polymer and put in a side group having at least six atoms linearly arranged, and having a terminal carboxylic acid or carbonyl halide group. Thus, hydroxyl containing polymers are suitable and preferred. The polymers may be organic polymers having polyolefin chains such as polystyrene, polyacrylates, polymethacrylates and polyvinyl alcohol. They may be polydiolefins, or condensation polymers such as polyamides, polyesters, polyurethanes, or polyamide-imides. They may be other types of polymers such as polypeptides, cellulose, polysaccharides and the like. They may

be inorganic polymers such as silica gel.

The linkage formed between the polymer and the initial nucleoside units is an ester-type linkage, using a predetermined hydroxyl group of the nucleoside and the acid group present on the side chain of the derivatized polymer. Thus in the case of a polyhydroxy polymer such as cellulose, polyvinyl alcohol or silica gel, which may be represented as:

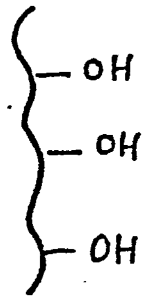

the first step is suitably to derivatize the polymer via the hydroxyls. Known methods can be adopted for this, and exemplary ones are described below, it being understood that these are not limiting. One such method is to react the polymer with -aminopropyltriethoxysilane, $NH_2(CH_2)_3Si(OEt)_3$ to form amine terminated side groups on the polymer:

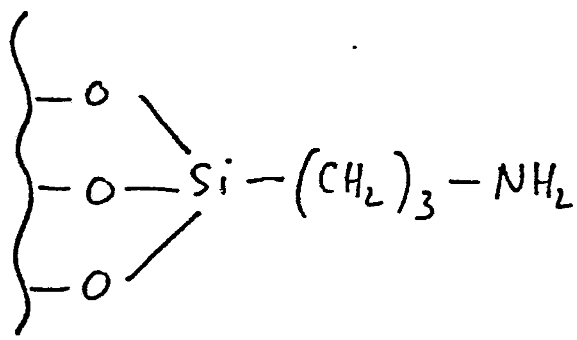

This may be reacted with a difunctional acid compound, e.g. succinic anhydride, to form the required acid group terminated polymer side chains with intermediate amide chain linkage, thus:

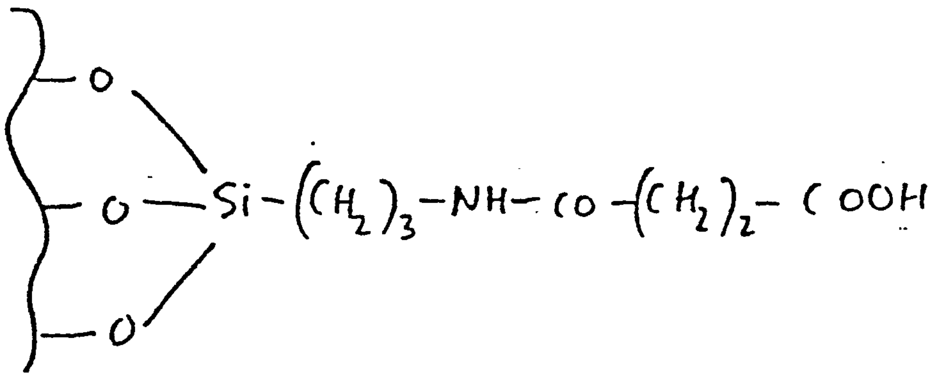

Another such method is the reaction of the hydroxyl groups with a half-ester of a dicarboxylic acid, for example, adipic acid half ester, pimelic acid half ester, suberic acid half ester, azelaic acid half ester or sebacic acid half ester. Then, after hydrolysis to convert the residual ester groups back to acid groups, the required derivatized polymer with acid terminated side groups of suitable chain length, and containing an ester linkage is formed.

A further alternative reaction is to treat the hydroxyl bearing polymer with a shorter chain diacid or diacid anhydride, e.g. succinic anhydride, followed by reaction with a polyalcohol of suitable chain length, thereby forming a polymer derivative having side chains of suitable length for use in the present invention, with intermediate ester linkages, and terminal hydroxyl groups. Subsequent treatment with succinic anhydride, for example, puts on the required acid terminal group.

In another alternative method, a hydroxyl-bearing polymer may be treated with a halobenzyl acid or ester compound, to produce a polymer having an ether linkage in the side chain,

thus:

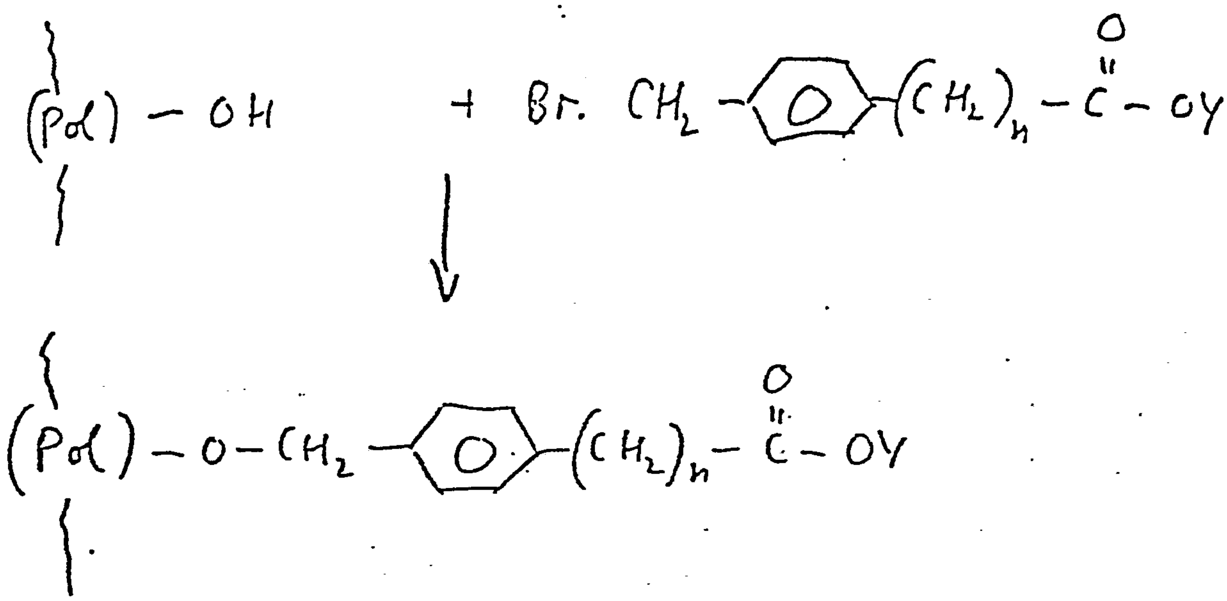

where n is an integer from 1-10. The product can now, or after appropriate hydrolysis, be reacted with the first blocked nucleotide. Instead of the halobenzyl acid or ester, there can be used a halo-trityl acid or ester, e.g.

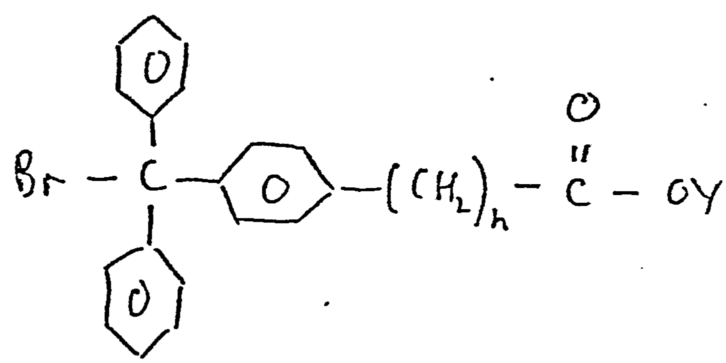

with substantially similar results.

It is not necessary to start with hydroxyl-bearing

polymers in the process of the present invention, although these are most suitable. It is, however, necessary that the polymer be capable of derivatization to put on a group -X-COOH or -X-CO-Hal. Polystyrene is a preferred, non-hydroxyl polymer, since it can be obtained with a suitable degree of cross-linking to obtain the required surface impermeability. Methods of derivatizing polystyrene and similar aromatic side group bearing polymers, to put acid or halocarbonyl groups thereon are known. One such method involves a Friedel-Craft reaction using aluminum trichloride and succinic anhydride, resulting in a polymer having the group -X-COOH attached to a side aromatic group.

In the next step of the process of the present invention, a nucleoside is preferably linked to the polymer at the 3' position on the nucleoside. The hydroxyl group at the 5' position on a nucleoside is more reactive than that at the 3' position. In order to maximize yields of product, and cut down reaction times, in a synthesis involving multiple chain extension steps, it is thus of advantage to react at the 5' position on the nucleotide as many times as possible, in preference to the 3' position. Since, in the process of the present invention, the link between the polymer and the initial nucleoside unit is to remain undisturbed throughout the chain extension steps, it is of advantage to use the 3' position for this link, so that the subsequent chain extension step can use the more reactive 5' hydroxyl and hence increase the yield of desired product.

For this purpose, the derivatized polymer is reacted

with a nucleoside having a free hydroxyl group at position 3' only, the hydroxyl at position 5' (and, in the case of ribonucleosides, the hydroxyl at position 2' also) being blocked:

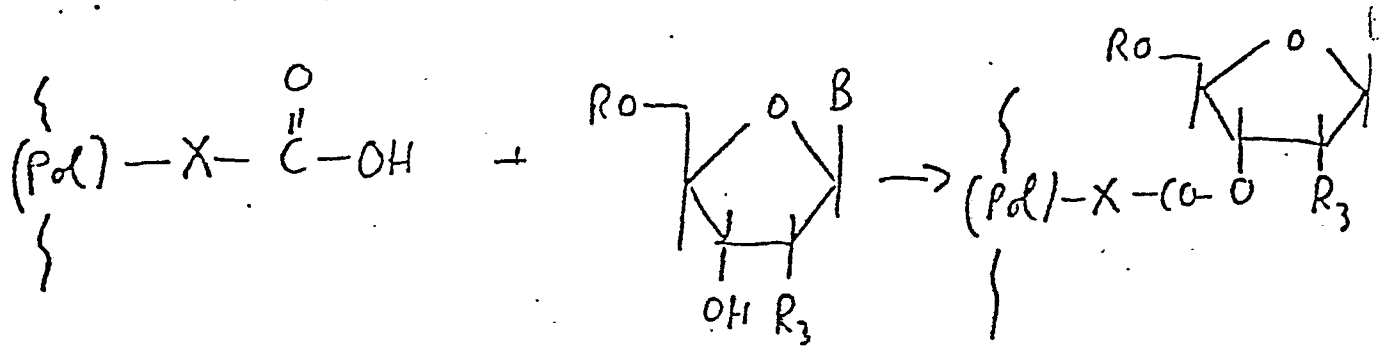

(VII) (VIII)

where -Pol- represents the polymer chain, R represents a blocking group such as methoxytrityl, B represents a base unit as previously described, selected from thymine, guanine, uracil, cytosine and adenine, $R_3$ represents H or a blocked hydroxyl group, and X is as previously defined. The reaction itself is well known, and is suitably accomplished in the presence of dicyclohexylcarbodiimide, DCC. Compound VIII, in its blocked or unblocked form, is a novel product and constitutes a significant feature of the present invention. When synthesizing polyribonucleotides, either the 2'-position or the 3'-position can be used to couple to the polymer, the other being blocked. Eventually, the polynucleotide is cleaved from the polymer and unblocked to leave hydroxyls at both 3' and 2' positions on this terminal unit, and so it is immaterial which is initially used for polymer coupling.

Compound VIII, in its blocked or unblocked form, has

additional utility, besides its use in polynucleotide synthesis as discussed. It is a solid, polymeric material, insoluble in aqueous and other simple solvents commonly used in organic synthesis, and has reactive side groups, namely reactive hydroxyl groups. It thus shows advantageous utility as a separation medium for separation of mixed solutions of organic or inorganic reactive products. For example, it can be used as the solid packing medium of a solvent separation column, down which mixed reagent solutions are poured for separation purposes, certain dissolved elements thereby being separated out on the column, for subsequent recovery in the known way.

The next stage of reaction according to the present invention is to react with a coupling agent at position 5', preparatory to adding another nucleoside unit to the chain. Firstly, the blocking group R is removed by conventional means (mild alkali), leaving the blocking group, in the case of a riboside, at position 2' in place, and then reacted with blocked phosphodichloridite:

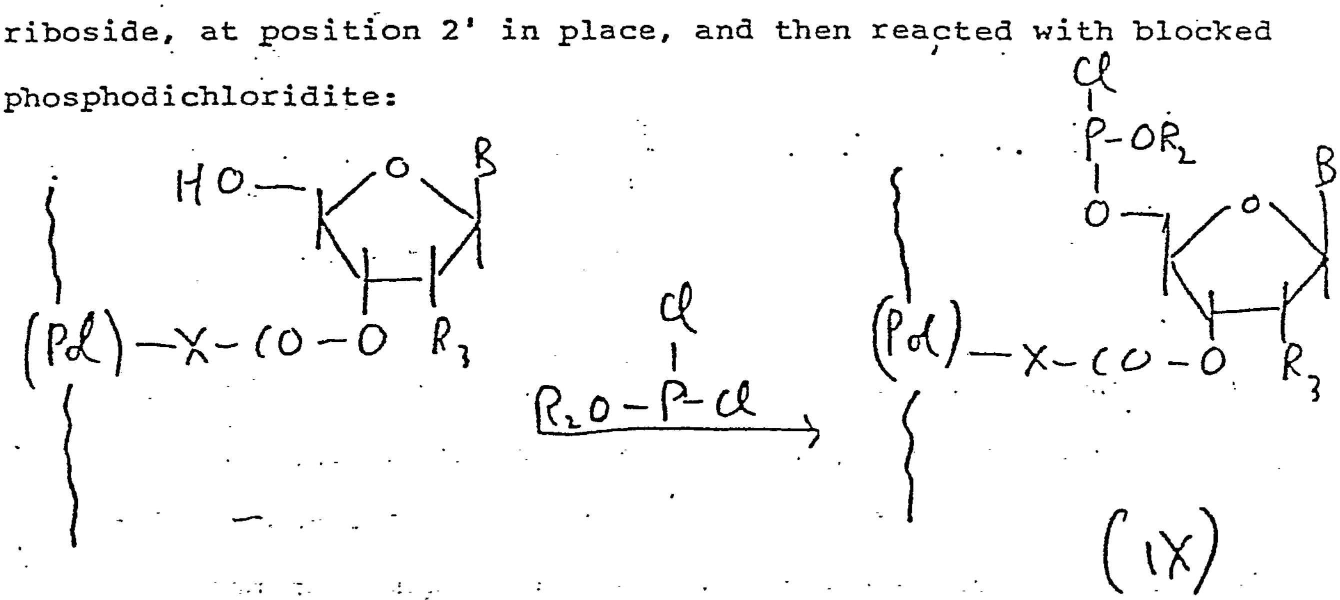

(IX)

In accordance with a preferred aspect of the present invention, group $R_2$ is methyl.

Then compound IX is reacted with a nucleoside unit blocked at its 5' position (and also, in the case of a ribonucleoside, at its 2' position) to form a 3', 5' linked chain of two units still linked to the polymer through X and an ester linkage:

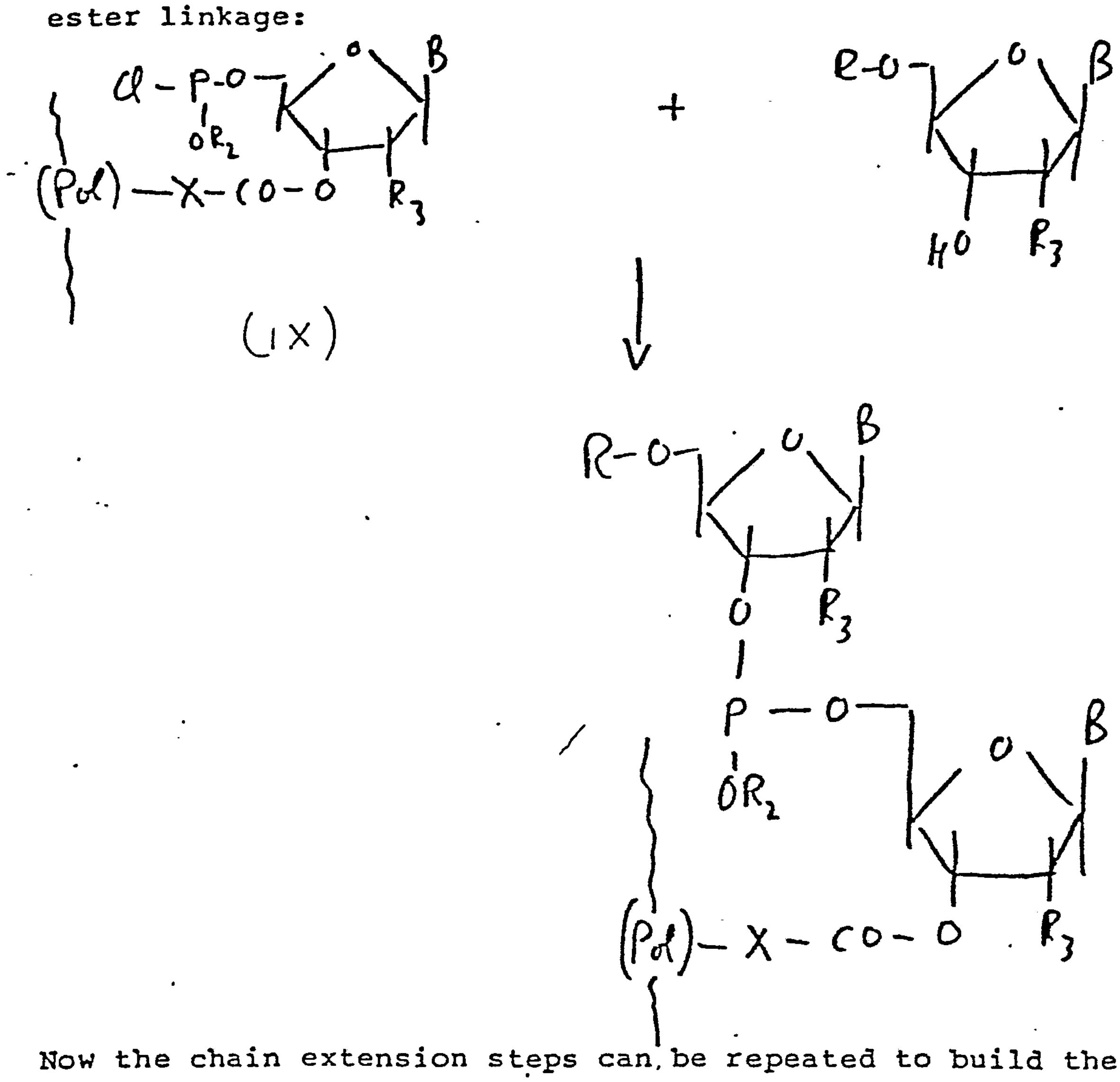

(IX)

Now the chain extension steps can be repeated to build the chain of desired length and predetermined sequence of units.

In the preferred process according to the invention, the nucleoside compound, blocked at the 5' position but

unprotected at the 3' position, and the blocked phosphodichloridite are pre-reacted, prior to their reaction with the polymer-nucleoside complex, thus:

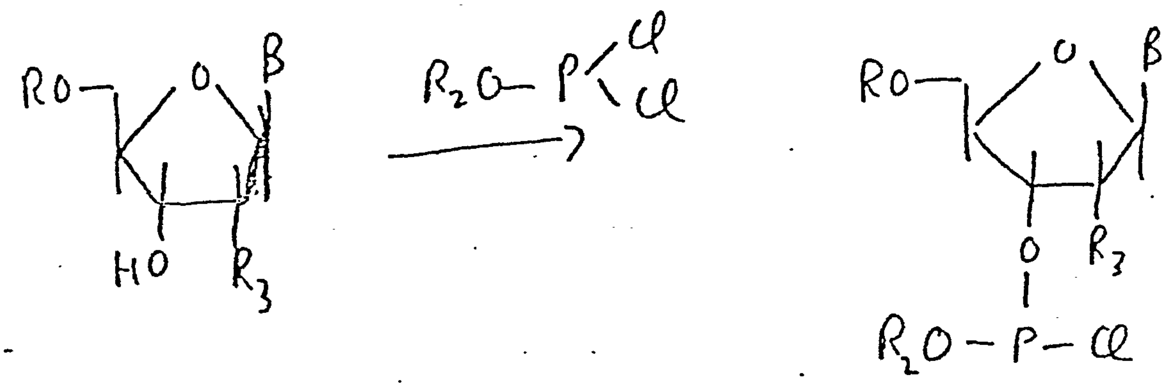

(X)

The preparation of compounds (X) leads to a simpler synthetic process, with higher yields of desired product, since it reduces the total overall number of synthetic steps to which the polymer-nucleoside complex must be subjected, with consequent reduction in the chances of undesirable side reactions involving the polymer complex. The blocked phosphodichloridite group at position 3' also obviates the need for a blocking group at that position and its subsequent removal at other stages. Moreover, reagents (X) after preparation can be readily purified and freed from starting materials prior to use. In conducting the above reaction using a riboside $R_3$ must of course represent a blocking group. The blocking group can then be left in place, until the stepwise synthesis is complete.

In one embodiment of the process, all chain extension steps are conducted with a reagent corresponding to formula (VII). In another embodiment, all chain extension steps are

conducted with a reagent corresponding to formula (X). In either case, therefore, stock bench reagents of formula VII or formula X, each with the same blocking group, can be prepared in advance and stored, with a storage facility for each of the four possible such reagents (ribo or deoxyribo based). Each can then be delivered as required to a reaction vessel, to conduct the process in the predetermined sequence. Thus, according to the invention, a semi-automated process may be used, with reagent storage vessels connected to a reaction vessel, and programmed to deliver a predetermined amount of selected reagent to the reaction vessel as and when required to build up a polynucleotide chain of the desired sequence. The reaction method according to the present invention allows this to be done, by reducing the number of different reagents required to a relatively small, handleable number in such a scheme. In a particularly preferred manner, the polymer-linked material, being solid and insoluble in the reagents used, can be contained in a column, and the reactions conducted by circulating the reagents through the column under the appropriate conditions.

Removal of the blocking groups at positions 5', at the various steps in the synthesis, is best accomplished with acetic acid in methanol. Whilst other reagents are known for this purpose, this reagent is preferred since it does not risk cleavage of the preformed chains.

A further advantage of the "solid phase", polymer based polynucleotide process of the present invention relates to the increased selectivity of the process. All of the hydroxyl groups

on the polymer may not be converted to acid functions in the polymer derivatization step. The derivatized polymer may contain other functional groups when it is reacted with the nucleoside, leading to byproduct formation. Moreover, all of the acid functions of the derivatized polymer may not react with the blocked nucleoside, at the first reaction stage, in the desired manner. Some acid and hydroxyl functions will inevitably remain, perhaps to react with the coupling agent, a nucleoside or a nucleotide reagent during a subsequent step. The result of such side reactions may well be a nucleoside-polymer complex, but with the wrong nucleoside group at the α position. As the chain extension reactions continue, this complex will also chain extend, but to give a polynucleotide with a slightly incorrect sequence. Such a product is unwanted and may in fact be potentially disastrous in its effects on end use, but is extremely difficult to separate from the desired product because of its chemical and physical similarity thereto.

The process of the invention may therefore include the additional step of "capping" the unreacted hydroxyl groups on the derivatized polymer, to prevent their unwanted participation in subsequent reaction steps. This can be done by treating the modified polymer with a suitable chemical reagent, such as phenylisocyanate, t-butyl-dimethylsilyl chloride or a similar alkyl silyl halide. In practice it is found that the residual hydroxyl groups are not very reactive in the subsequent reaction stages, so that the hydroxyl capping of the derivatized polymer may not be essential except as an ultimate safeguard.

The present of residual unreacted acid groups on the modified polymer, after reaction with the first nucleoside unit, is however detrimental to the subsequent chain extension steps. There is a strong possibility that these will lead to the formation of incorrect nucleotide chain sequences. Moreover, their presence tends to interfere in other ways with the carrying out of subsequent chemical reactions on the modified polymer-nucleoside complex. The preferred process of the present invention therefore includes the step of capping residual, unreacted acid or acid halide functions on the modified polymer, after reaction thereof with the initial nucleoside unit. This is suitably accomplished by reaction with alcohol, or other known reagents for treating acid groups. In the most preferred semi-automatic process of the invention, the polymer derivatization, coupling with first nucleoside unit, and residual acid groups capping takes place prior to introduction of the intermediate product into the column.

Additional possibilities for side reactions will occur after each step of reaction of an intermediate product with phosphodichloridite or with phosphordichloridite-nucleoside complex. In each case, an intermediate polymer-linked nucleoside or nucleotide, the end unit of which has an unblocked hydroxyl group at its 5' position, is reacted with blocked phosphodichloridite or a phosphodichloridite-nucleoside reactant (X). 100% reaction therebetween will not occur so that the resulting product mixture will contain some hydroxy-terminated, polymer linked nucleotide capable of forming unwanted

polymer-linked byproducts in subsequent reaction steps.

The process of the present invention thus may include, after each phosphodichloridite-polymer-linked nucleoside reaction synthesis step an additional capping reaction step, in which all or substantially all the residual unreacted hydroxyl groups are rendered inactive, prior to proceeding to the next step of the synthesis. By adoption of such "capping" procedures, the likelihood of growth of unwanted polynucleotide polymer complexes is effectively eliminated. These intermediate capping steps are, however, optional, since in practice it is found that the individual chain extension steps proceed to such high conversions that only small amounts of residual hydroxyl is left after each chain extension step. Moreover, any such byproduct will differ from the desired product in having too few nucleotide units, so that its separation from the desired product is not unduly difficult. On occasion, however, it is desirable to conduct such capping reaction, especially if one wants to recover a "byproduct" of known nucleotide chain length and base sequence as well as the predetermined product. The process of the present invention allows this to be done. Suitable reagents for use in capping the residual hydroxyl are those referred to above, as useful for treating the polymer.

In the preferred process of the invention, phosphodichloridite is used as the chain extending coupling agent, preferably pre-reacted onto the nucleoside, although the process of the invention is not limited thereto. Alternatives are the known processes using tetrazole condensing agents, and

using nitro-imidazole condensing agents. Phosphodichloridite has been found to give the most satisfactory yields of coupled product at each stage with smaller amounts of side reaction. Moreover, one does not need to block the amine group on the base B, when using phosphodichloridite. Its hydroxyl group is protected during chain extension reactions, preferably by use of a methyl blocking group, but if desired protected by a suitable alternative protecting group such as trichloroethyl, chlorophenyl, cyanoethyl and tribromoethyl. The phosphite blocking groups are left in position until all the required chain extension steps have been completed, since their blocking function is required at all stages, and then they are all removed at the same time.

Also to complete the synthesis of a polynucleotide, when using phosphodichloridite as linking reagent, the phosphite groups linking the nucleoside units must be oxidized to phosphate groups. In the process of the present invention, this may be done before or after the polynucleotide chains are stripped from the polymer base support. A suitable oxidation process, as is known, involves reaction with iodine and water. It is preferred to conduct this oxidation step whilst the polynucleotide chains are still attached to the polymer, and whilst the hydroxyl group on the phosphite linkage is still blocked by its appropriate protecting group. It is a very significant feature of the present invention that all of the blocked phosphite groups can be oxidized to phosphate in a single stage, after all chain extension steps have been

completed, rather than individual oxidation of phosphite to phosphate at each stage of the chain extension. This results in a very considerable saving of time and process steps, with consequent reduction in the possibilities of side reactions and increase in ultimate yields of desired product.

The removal of the blocking group from the phosphite/phosphate is preferably performed before the polynucleotide chains are stripped off the polymer. In this way, the reagents used to detach the polynucleotide chains from the polymer (normally $^{-}OH$) will not attack the phosphite/phosphate group. In the case of methyl protecting groups, in accordance with the second aspect of the invention, these can be simply and neatly removed by very mild treatment with tertiarybutylamine. The boiling point of tertiarybutylamine is $40^{o}C$, so that the product can be reacted under reflux therewith to remove the methyl group from the phosphite/phosphate, without risk of cleaving the polynucleotide chain. Moreover, tertiarybutylamine is non-toxic, so that its residues are not harmful. With such a low boiling point, in fact, residues of tertiarybutylamine can be boiled off without risking chain cleavage. Other blocking groups than methyl need to be removed with metals such as zinc and copper, which is much more difficult and unreliable, and may lead to metal contamination of the final product. Instead of tertiarybutylamine one may use ammonium hydroxide which besides removing the methyl blocking group will also cleave the polynucleotide chains from the polymer.

In the preferred method of the invention, therefore, immediately prior to removal of the product from the polymer chain but after oxidation to phosphate, one has a product of the following general chemical formula:

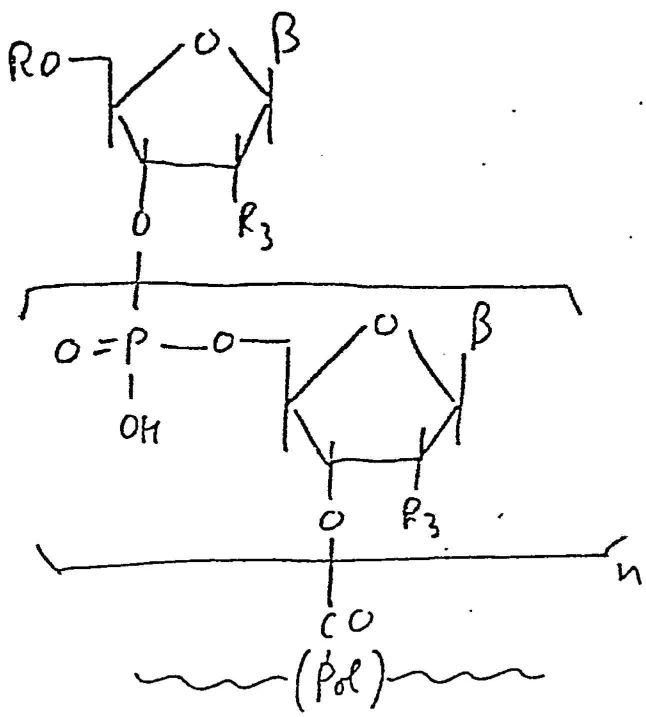

To complete the synthesis of the polynucleotide, it is now necessary to strip the polynucleotide chains from the polymer, and remove terminal blocking group R from the 5' position of the terminal nucleoside unit. These two steps can be conducted in either order, but it is preferred to strip from the polymer first so that a vulnerable chain-terminal hydroxyl group is not prematurely introduced. In point of fact both of these steps may if desired by conducted simultaneously, using a reagent such as ammonia or other OH reagent, provided a suitable initial choice of blocking group R has been made. The bond CO-O to be cleaved on stripping the polynucleotide from the polymer and removing the blocking group R is much weaker than the P-O

bonds through which nucleotide units are coupled in the polynucleotide chain.

Finally the product may be purified and separated from side reaction products, e.g. by conventional chromatographic column methods.

The basic steps of a preferred polynucleotide synthesis method according to the present invention may be summarized as follows:

1. Treatment of polymer to put on desired reactive side groups.

2. Reaction of polymer with 5' blocked nucleoside VII to obtain product VIII.

3. Treatment of the reaction product to deactivate residual reactive acid groups on the polymer.

4. Removal of 5' blocking groups from product VIII.

5. Reaction of unblocked product VIII with blocked phosphodichloridite-nucleoside complex reagent X.

6. Repetition of steps 4 and 5 as desired to extend the polynucleotide chain in the desired sequence.

7. Oxidation of the phosphite groups to phosphate and unblocking of phosphate.

8. Stripping of the polynucleotide chains from the polymer.

9. Removal of terminal 5' nucleoside blocking group.

If methyl is used to block the phosphite/phosphate groups, as is preferred, then the unblocking of the phosphate

and the stripping from the polymer may be accomplished in a single step, using ammonium hydroxide as the reagent.

The synthetic method of the present invention thus has considerable advantages of simplicity, and reduction of the number of reaction steps, especially where long chain polynucleotides are to be made. Reagent separation and byproduct formation has been very significantly reduced, and effectively eliminated as a repetitious step to be conducted on all intermediate products. Yields of desired product are significantly increased. The synthetic method makes use of a small number of standard reagents.

The process of the present invention is applicable, as noted previously, to the preparation of either deoxyribopolynucleotides or ribopolynucleotides. These units differ from one another in chemical structure 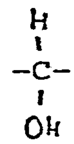 at position 2', whereas the deoxyribo unit has a grouping of 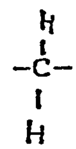 at position 2' when synthesizing ribopolynucleotides therefore, the 2'-hydroxyl group must be appropriately blocked to ensure the correct 3'-5' coupling. Useful blocking groups for this purpose are well-known in the art, and include t-butyldimethylsilyl, and similar such groups to be found disclosed in the prior art. The blocking group chosen for position 2' on the ribonucleotide or ribonucleoside units should be different from that chosen for position 5', so that the individual blocking groups can be selectively removed.

The use of alkaline conditions in the synthesis of polyribonucleotides should be avoided since RNA chains are alkali labile. In particular, $^{-}OH^{2}$ ions are known to interact with the phosphate groups linking RNA chain units, with the result of hydrolysing the -P-O- linkage and causing chain cleavage. Such reaction must of course be avoided during RNA synthesis according to the present invention.

The accompanying Figure illustrates diagrammatically an apparatus for carrying out the process of the invention, on a semi-automatic scale. A column 10 is provided in which is a mass 14 of polymer-initial nucleoside unit compound (VIII), prepared exteriorly of the column and treated with primary aliphatic alcohol to cap residual acid functions, supported on a perforated plate 12. The column 10 has a heat insulation jacket 16. The column is equipped with a recirculation line 18. The outlet from the bottom of the column 10 is via a two way valve 20 to permit liquid from the column 10 to exit via outlet 22 or recirculate via recirculation line 18.

The main inlet 24 to the top of the column 10 is via a pump 26 from a manifold 28 having a multi-position distributor valve therein by means of which it may be arranged to pass to the column 10 reagents from any one of seven reagent storage vessels via their respective outlet pipes, or from the recirculation line 18. First vessel 30 contains a solution of the blocked 5'-nucleoside compound VII, namely 5'-dimethoxtrityldeoxyguanosine, pre-reacted with and coupled to blocked phosphodichloridite (compound X) in pyridine solvent and admixed

with condensing agent dicyclohexylcarbodiimide in predetermined correct preparation for reaction. Second vessel 32 contains a similar solution and mixture of 5'-dimethoxytrityldeoxythymidine coupled to blocked phosphodichloridite. Third vessel 34 contains a similar solution and mixture of 5'-dimethoxytritylcytidine coupled to blocked phosphodichloridite. Fourth vessel 36 contains a similar solution and mixture of 5'-dimethoxytrityladenosine coupled to blocked phosphodichloridite. Fifth vessel 38 contains pyridine solvent, for washing between each reaction step. Sixth vessel 40 contains acetic acid in methanol or acetonitrile for use in unblocking the 5' position of the last nucleotide unit coupled to the forming product. Seventh vessel 42 contains phenylisocyanate, for optional use in capping off unreacted hydroxyl groups remaining after a chain extension step.

The apparatus includes a microprocessor 44 controlling the manifold valve 28, outlet valve 20 from the column 10 to outlet 22 and recirculation line 18 and pump 26. Microprocessor 44 is programmed, to supply reagents at the required times and in the required amounts to the column 10 to react with the polymer-linked nucleotide therein, to recirculate them as required, etc. Thus, a polynucleotide of any desired unit sequence can be prepared semi-automatically, by correct programming to conduct the previously described reaction steps in sequence. After the chain length has been built as desired, the phosphite oxidation and unblocking can be conducted in the column, and the polynucleotide cleaved from the polymer either

in the column or after extraction therefrom.

EXAMPLE 1

In this example according to the invention, a polynucleotide was prepared on a modified silica gel polymer.

A 20gm sample of silica gel 70-230 mesh ASTM was refluxed for four hours with 100 ml concentrated hydrochloric acid, cooled, filtered, washed until neutral pH obtained, and then dried overnight under high vacuum, to obtain 18.65g of dry, treated silica gel polymer. Next, the silica gel polymer was derivatized, to put appropriate side groups -X-COY thereon, for subsequent reaction with the nucleotide units.

For this purpose, the 18.65g sample of silica gel was refluxed, under stirring, with 10ml of 3-aminopropyltrimethoxysilane and 100 ml of anhydrous toluene. Ethanol containing toluene was removed from the refluxing mixture periodically. The total reflux time was four hours, after which the reaction mixture was cooled and filtered, and the solid component washed with toluene, ether and then dried. 21.8g of product amino-silica gel was obtained. This product was next suspended in 200 ml water and stirred at room temperature. Over a period of one hour, 20g of succinic anhydride was added dropwise, the suspension being maintained at pH4 by sodium hydroxide addition. After stirring at room temperature for about two hours, the residual succinate was washed out and the derivatized silica gel dried under vacuum at 40°C overnight. 22.1g of product was obtained.

In order to cap off any remaining hydroxyl groups on

the derivatized silica gel, it was next reacted with 10 ml trimethylchlorosilane, in 60 ml anhydrous pyridine, under cooling, at 25°C. After reaction for eight hours, the suspension was filtered and the solid washed and dried under vacuum overnight. 23.85g of derivatized, capped silica gel was thus obtained.

Next the derivatized silica gel so prepared was reacted with a 5'-blocked nucleotide unit, to couple the nucleotide to the polymer support by reaction of its 3' hydroxyl group with terminal carboxyl groups on the polymer side chain. Thus, 1.6 grams 1'-guanine-5'-dimethoxytrityl-deoxyribose (5'-dimethoxy-trityl-deoxyguanosine) was condensed with 10g of the derivatized silica gel in anhydrous pyridine using dicyclohexylcarbodiimide (5.1g) as condensing agent, under careful stirring for 40 hours. Then residual acid groups on the modified polymer were capped by conversion to inert amide, using addition first of p-nitrophenol, allowing to stand overnight, washing with pyridine and then adding piperidine. After filtering under nitrogen and washing, 9.0g of solid product was obtained.

The product was next treated with 25 ml of 80% acetic acid, 3 parts, mixed with 1 part methanol by stirring at room temperature for about 1 hour to remove the dimethoxytrityl blocking group from the 5' position of the nucleoside linked to the polymer. After this treatment, the product was filtered, washed with pyridine and subsequently with ether, and dried at high vacuum. For chain extension, the product was next reacted with methyl phosphodichloridite nucleoside complex.

For this step, collidine (1.060 mls) and THF (3ml) were mixed and cooled to -78°C. 186.2mg of methyl phosphodichloridite was added promptly and mixture stirred. Then there was added nucleoside 1'-thymine-5'-dimethoxy-

trityldeoxyribose (5'dimethoxytrityl-deoxythymidine) in the amount of 952 mg and 3ml THF, dropwise, and stirred for five minutes. This pre-formed the nucleoside-phosphodichloridite complex. Next 8.9g of the silica gel-nucleoside, along with another 5ml THF were added, the mixture stirred for one hour, warmed to -10°C and treated with a THF/water mixture. The precipitate was filtered off, and washed several times. The solid reaction product was then treated with phenylisocyanate in pyridine, to cap off any residual unreacted hydroxyl groups.

The de-tritylation step was next repeated to unblock the 5' position of the terminal nucleoside unit. 8.5g of the solid product was treated with 25ml of 80% acetic acid, 3 parts, in methanol, 1 part as before, to yield 7.48gm of solid product for the next reaction stage.

The chain extension step was repeated as before, to form a trimer. In place, of 5'-dimethoxytrityl-deoxythymidine, there was used 5'-dimethoxytrityl-deoxy-cytidine (950.4mg). The resulting product was capped by treatment with phenylisocyanate in pyridine.

The experiment was continued as before, to obtain a tetramer. Thus the steps of unblocking, chain extension and recovery were repeated, in this instance using 5'-dimethoxy-trityl-deoxythymidine as the blocked nucleoside. Capping with phenylisocyanate was again undertaken, after the chain extension reaction. The polymer-linked tetramer was chain extended to a pentamer, using 5'-dimethoxytrityl-deoxythymidine and then to a hexamer using 5'-dimethoxytrityl-deoxy-cytosine,

using the unblocking, chain extending, recovering and capping steps described above. Subsequently, the hexamer was oxidized to phosphate, in a single step for all phosphite linkages, and treated successively with acid for detritylation and ammonia to strip from the polymer and remove remaining protecting groups. Over 70% conversion was obtained at each step, to give a final hexamer yield of about 20%, based on the amount of nucleoside originally attached to the polymer.

The above procedure has been essentially repeated, to prepare octamer and hexamer polynucleotides, with various predetermined sequences of bases, with essentially similar or even improved results.

EXAMPLE 2

In this example, a polynucleotide was prepared by coupling nucleotide units to a modified solid polymer, namely silica, contained in a column, and passing the various reagents down the polymer-filled column. The apparatus comprised a column in which the polymeric material was packed, without any additional support material, inlet and outlet tubes for the column and a pump to arrange recirculation of liquid reagents through the column.

The polymer was prepared and derivatized, as previously described, to give side chain terminal carboxy groups. In a flask, the dried, derivatized polymer was condensed with 1-thymine-5'-dimeth-

oxytrityl deoxyribose, and then detritylated with acetic acid-methanol. The solid polymeric product material was separated, washed and packed into the reaction column. The next nucleotide unit was prepared by reacting 1'-thymine-5'-dimethoxytrityldeoxyribose (550 mg) with methyl phosphodichloridite (78 ml) in pyridine (50 ml) at $0^{o}C$ on a salt-ice bath. The resulting mixture was then circulated through the column at a flow rate of 2 ml per minute, for one hour, at room temperature and at a pressure of about 1000 psi. Then, 300 mg of iodine

dissolved in 40:10 tetrahydrofuran was circulated through the system at 3 mls per minute for 30 minutes, to oxidize the phosphite group to phosphate. The column was washed with acetonitrile at room temperature and the contents extracted. Thin layer chromatography indicated coupling yields of the second nucleotide of the order of 60-70%. This experiment demonstrates the high yields and simplicity of a solid phase, semi-automatic process according to the invention, adaptable to produce polynucleotides on a large, efficient scale.

EXAMPLE 3

In this Example, a polyribonucleotide was prepared by procedures as previously described.

The polymer support used was Vydak TP silica, a particularly suitable rigid non-swellable polymer. The polymer was first activated by refluxing HCl and then derivatized successively with 3-aminopropyltriethoxysilane, succinic anhydride and trimethylchlorosilane, as previously described. The derivatized polymer, 6g, was treated with 5'-monomethoxytrityl(-3'-*t*-butyldimethylsilyl)-guanosine (0.005 m. mole/g), DCC (4.6g) and 4-dimethyl-aminopyridine (DAP 146 mg) in pyridine (150 ml) with shaking for 48 hours. This produced a ribonucleoside linked at position 2' to the polymer, TBDMS-protected at position 3' and MMT-protected at position 5'. The MMT group was removed with 0.05N benzene sulfonic acid (20 min) in acetonitrile. The amount of nucleoside bound to the polymer was found to be 0.16 m mole.

To 3g of the polymer-nucleoside complex was added 0.66 m mole of 5'-O-monomethoxytrityl-2'-t-butyldimethylsilylcytidine which had been previously treated with 0.59 m mole of methyldichlorophosphite in THF (5 ml) containing collidine (0.4 ml) for 15 min. at -78°C. The mixture was stirred at -78°C for 30 min., and allowed to warm to room temperature (total 30 min.). The phosphite linkage was oxidized to the phosphate by stirring with iodine/water for 10 min at room temperature. The polymer was washed with methylene chloride (15 ml) and ether (10 ml) and dried. A sample (100 mg) of the polymer was treated successively with 0.05 N BSA for 20 min. and conc. $NH_4OH$/pyridine (2:1) for 14 h. The solution was collected by filtration, concentrated at reduced pressure and the residue was treated with tetrabutylammonium fluoride in THF for 90 min. The solution was passed through a Dowex 50WX8 $Na^+$ column, concentrated and applied to Whatmann 3 mm paper developed in Solvent F (n-propanol-conc. $NH_4OH$-water (55:10:35) ). The yield of CpG (90%) was determined spectrophotometrically as % conversion.

The remainder of the polymer (2.9g) was treated with excess phenylisocyanate in pyridine for 2 h and was then detritylated with BSA and dried in preparation for the next coupling step. All subsequent coupling steps were carried out as described above and the % conversions and properties of the oligonucleotides are listed in the following Table and range from 85 to 98%

1056 (1977) ). The products can similarly be used to make insulin, from appropriate cells, modifying the DNA therein with products made according to the process of the invention (Goeddel et al, Proc. Natl. Acad. Sci USA, 76, 106 1979).

0040099

Table

| Compound | $R_m^{Tp}$ | $R_f^F$ | Yield (%) |
|---|---|---|---|
| CpG | 0.40 | 0.39 | 90 |
| UpCpG | 0.44 | 0.34 | 98 |
| CpUpCpG | 0.59 | 0.30 | 85 |
| GpCpUpCpG | 0.66 | 0.25 | 87 |
| ApGpCpUpCpG | 0.76 | 0.18 | 86 |

The tetranucleotide CpUpCpG was degraded with RNAse A to Cp, Up and G in the ratio 2.03:0.96:1.0. The hexanucleotide ApGpCpUpCpG was degraded with RNAse $T_2$ to Ap, Gp, Cp, Up and G in the correct ratios. Simultaneous treatment with snake venom and phosphatases converted the hexamer to C, U, G and A in the correct ratios.

The ribonucleotide hexamer so prepared corresponds to units 21-26 of E. Coli t $RNA_f^{Met}$. It contains at least one unit corresponding to each of the four common ribonucleosides.

Polynucleotides prepared according to the process of the present invention are useful in a variety of biochemical and biological processes, where polynucleotides prepared by different processes have already shown utility. For example, the natural gene for preparation of a desired peptide product such as the mammalian hormone somatostatin can be synthesized as DNA fragments, assembled, joined to form double stranded DNA, and inserted into the DNA of bacterial cells, to produce somatostatin as a cell product (Itakwa et al, "Science" 198,

Claims:

1. A process for preparing polynucleotides of predetermined chain length and nucleotide unit sequence, which comprises:

(i) reacting a solid polymer which has side groups of formula -X-CO-Y in which Y represents hydroxyl, chloro, bromo or iodo and X represents a chemical group having at least 6 linearly arranged atoms therein, with a pre-selected partially blocked nucleoside of general formula

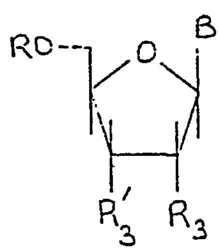

(A)

in which B represents a base selected from uracil thymine, guanine, adenine and cytosine, R represents a blocking group, and either $R_3'$ is hydroxyl and $R_3$ is hydrogen or blocked hydroxyl or $R_3'$ is blocked hydroxyl and $R_3$ is hydroxyl to form a polymer-nucleoside product coupled through an ester linkage;

(ii) reacting the polymer-nuceloside coupled product so formed to replace group RO at position 5' with a hydroxyl group;

(iii) reacting the 5' -unblocked polymer-nucleoside coupled product of step (ii) with a blocked phosphodichloridite and a

preselected, blocked nucleoside of general formula (A) given above, which blocked phosphodichloridite and nucleoside may have been prereacted;

(iv) repeating steps (ii) and (iii) above sequentially, a predetermined number of times, with preselected, blocked nucleosides of general formula (A) given above to yield a polymer-linked polynucleotide chain of predetermined unit sequence;

(v) oxidizing the polymer-polynucleotide product of step (iv) to convert phosphite groups therein to phosphate groups;

(vi) removing the residual blocking groups and reacting the product to cleave the ester linkage of the polynucleotide to the polymer, so as to recover a polynucleotide of predetermined unit sequence and chain length.

2. The process of claim 1 including the additional step of treating said reaction mixture from step (i) with acid-reacting groups so as to deactivate residual -X-COY groups on said polymer.

3. The process of claim 1 or 2 which includes the step of reacting together a blocked nucleoside of general formula A with blocked phosphodichloridite of general formula:

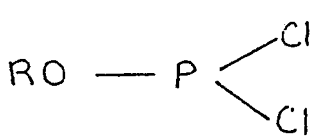

in which $R_2$ is a blocking group, to form a nucleoside-phosphodichloridite complex, and using said complex in step (iii) of the process.

4. The process of claim 1, 2 or 3 including the additional step of treating the reaction mixture from step 3 with hydroxyl-reacting groups so as to deactivate residual -OH groups at the 5' position of the terminal nucleoside unit and prevent reaction thereof in subsequent process steps.

5. The process of claim 4 wherein said treatment is conducted by reacting said reaction mixture with phenyl isocyanate or an alkyl silyl halide.

6. The process of any one of the preceding claims, wherein the blocking group of the phosphodichloride compound is a methyl group.

7. The process of claim 6 wherein the unblocking of the methyl group on the phosphodichloridite is conducted by treating the product with t-butylamine.

8. The process of any one of the preceding claims wherein said oxidation step (v ) is a single step conducted after termination of all of steps (ii) and (iii) to effect simultaneous oxidation of all phosphite groups in the polynucleotide chains.

9. A solid nucleoside-polymer complex having the general formula

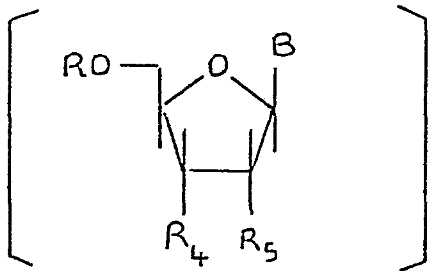

wherein one of $R_4$ and $R_5$ is an ester linkage to a solid polymer chain and having general formula

Pol-X-CO-O- .

in which Pol represents the solid polymer chain,

and X represents a chemical group having at least six lineraly arranged atoms therein and comprising carbon-carbon chains optionally interrupted by one or more linkages selected from the group consisting of ester linkages, amide linkages and ether linkages;

and the other of $R_4$ and $R_5$ is selected from hydrogen and blocked hydroxyl;

R represents a hydrogen or a hydroxyl blocking group; and

B represents a base selected from thymine, guanine, adenine, cytosine and uracil.

10. A solid nucleoside-polymer complex according to claim

9 having the general formula

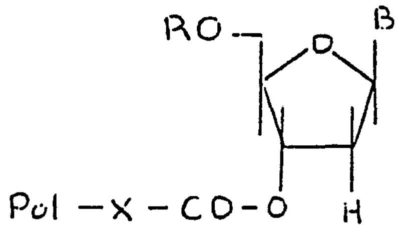

where -Pol • represents a polymer chain selected from the group consisting of Si-O chains, polyolefin chains, polydiolefin chains, polyester chains, polyamide chains, polyamide-imide chains, polyurethane chains, polypeptide chains, polysaccaride chains and polyether chains;

X represents a chemical group having at least six linearly arranged atoms therein and comprising carbon-carbon chains optionally interrupted by one or more linkages selected from the group consisting of ester linkages, amide linkages and ether linkages;

R represents hydrogen or a hydroxyl blocking group; and

B represents a base selected from thymine, guanine, adenine and cytosine.

11. A solid ribonucleoside polymer complex according to claim 9 wherein one of $R_4$ and $R_5$ is blocked hydroxyl and the other is said polymer-ester linkage, and B represents a base selected from uracil, adenine, guanine and cytosine.

12. A solid nucleoside-polymer complex according to claim 9, 10 or claim 11 wherein -Pol- represents a polymer of silica.

13. A solid nucleoside-polymer complex according to claim 9, 10 or claim 11 wherein -Pol- represents cellulose.

14. A solid nucleoside-polymer complex according to any one of claims 9 to 13 wherein X represents an aliphatic amido-containing group of formula $-(CH_2)_3-NH-CO-(CH_2)_2$.

15. A process of preparing a polynucleotide which comprises reacting a first nucleoside of general formula

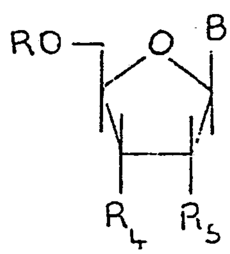

wherein R is selected from the group consisting of hydrogen and hydroxyl-protecting groups;

$R_4$ and $R_5$ are selected from the group consisting of hydrogen, hydoxyl protecting groups, and groups of formula Pol-X-CO-O wherein Pol represents a polymer chain selected from the group consisting of Si-O chains, polyolefin chains, polydiolefin chains, polyester chains, polyamide chains, polyamide-imide chains, polyurethane chains, polypeptide chains, polysaccharide chains and polyether chains; and X represents a chemical group having at least six linearly arranged atoms therein and consisting of carbon-carbon chains optionally interrupted by one or more ester, amide or ether linkages, with the proviso that one of $R_4$ and $R_5$ is said polymeric-linked group and B represent a base derivative

selected from the group consisting of thymine, guanine, adenine, cytosine and uracil, with a phosphodichloridite derivative having the general formula

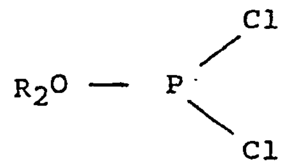

wherein $R_2$ is methyl or benzyl, to form a nucleoside compound with a second nucleoside meeting the general formula of said first nucleoside except that neither of $R_4$ and $R_5$ is said polymeric-linked group; and subsequently replacing group $R_2$ with H by reaction with tertiarybutylamine.

16. A process according to claim 15 wherein $R_2$ is methyl.

17. A process according to any one of claims 1 to 8, wherein the product of step (i) or the first step (ii) is provided in a column and at least the remaining steps (ii) and (iii) are carried out by passing reagents through the column in sequence.

18. A process according to claim 17, wherein the reagents are delivered to the column via a manifold provided with a multi-position distributor valve arranged to allow passage of reagents from any one of a

plurality of supply vessels.

19. A polynucleotide when prepared by the process of any one of claims 1 to 8, or 15 to 19.

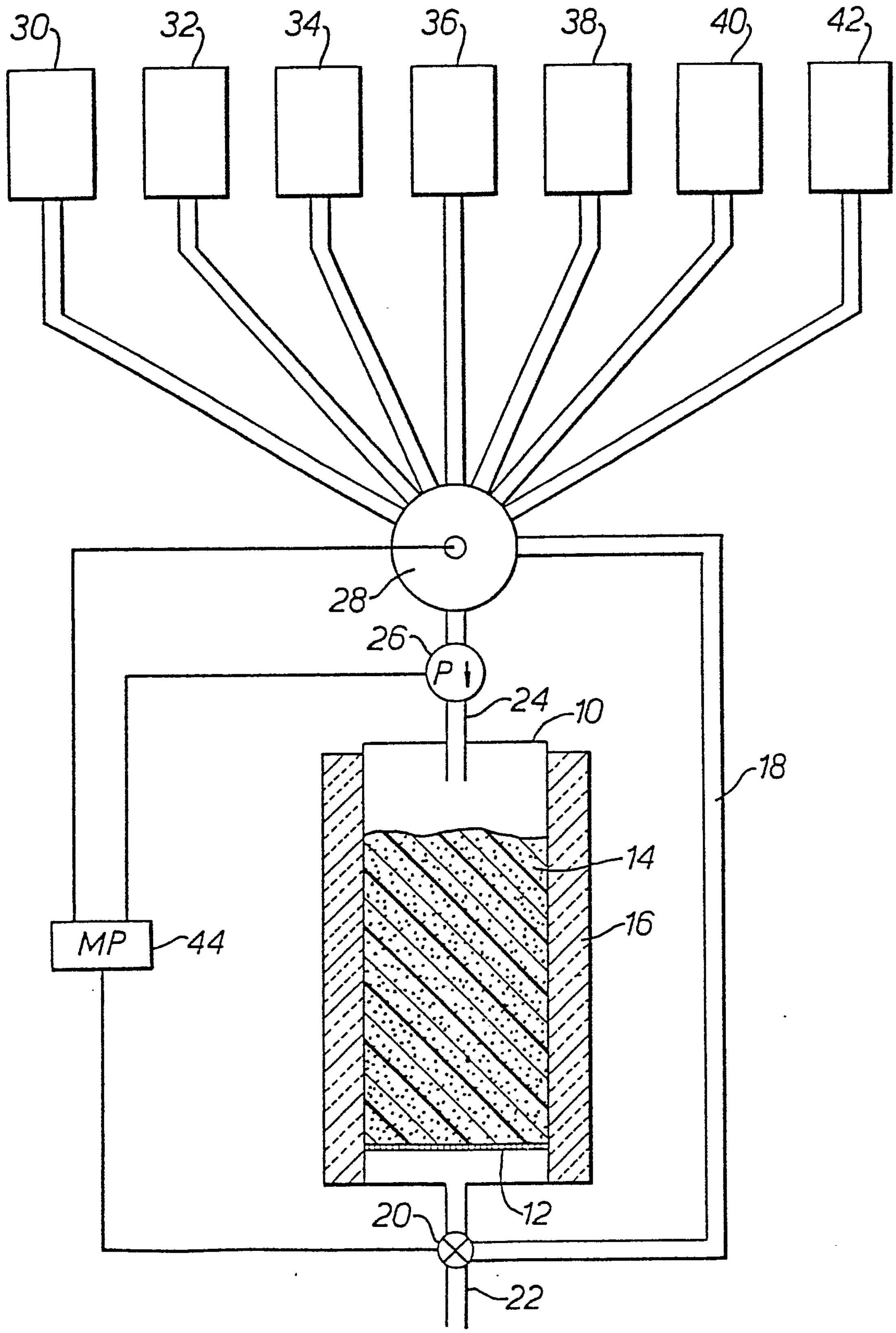
30
32
34
36
38
40
42
28
26
P
24
10
18
14
16
MP
44
20
12
22

0040099

European Patent Office

EUROPEAN SEARCH REPORT

Application number
EP 81 30 2110



| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | ADVANCES IN CARBOHYDRATE CHEMISTRY AND BIOCHEMISTRY, vol. 36, 1979, Academic Press, New York, Ed. R. STuart Tipson et al. M. IKEHARA et al. "The synthesis of polynucleotides", pages 135-213.<br>* Pages 135-213 *<br>-- | 1 | |
| | TETRAHEDRON LETTERS, vol. 21, no. 43, 1980 Pergamon Press, GB K.K. OGILVIE et al. "Silica gel as solid support in the synthesis of oligoribonucleotides" pages 4159-4162.<br>* Pages 4159-4162 *<br>-- | 1-19 | TECHNICAL FIELDS SEARCHED (Int. Cl.3) |
| | CANADIAN JOURNAL OF CHEMISTRY, vol. 58, no. 21, November 1, 1980, publ. by The National Research Council of Canada. K.K. OGILVIE et al. "The chemical synthesis of oligoribonucleotides. IX. A comparison of protecting groups in the dichloridite procedure", pages 2686-2693.<br>* Pages 2686-2687 *<br>----- | 6 | |

0040099

European Patent Office

# EUROPEAN SEARCH REPORT

Application number
EP 81 30 2110

| Category | Citation of document with indication, where appropriate, of relevant passages | Relevant to claim | CLASSIFICATION OF THE APPLICATION (Int. Cl.³) |
|---|---|---|---|
| X | TETRAHEDRON LETTERS, vol. 21, no. 8, 1980, Pergamon Press, GB M.D. MATTEUCCI et al. "The synthesis of oligodeoxypyrimidines on a polymer support" pages 719-722. * Pages 719-722 * -- | 1-19 | C 07 H 21/00 |
| | JOURNAL OF THE AMERICAN CHEMICAL SOCIETY, vol. 98, no. 12, June 9, 1976, R.L. LETSINGER et al. "Synthesis of thymidine oligonucleotides by phosphite triester intermediates" pages 3655- 3661. * Pages 3655-3661 * -- | 3 | TECHNICAL FIELDS SEARCHED (Int. Cl.³) C 07 H 21/00 |
| | JOURNAL OF THE AMERICAN CHEMICAL SOCIETY, vol. 101, no. 22, October 24, 1979 P. TUNDO et al. "Synthesis, catalytic activity, and behavior of phase-transfer catalysts supported on silica gel. Strong influence of substrate adsorption on the polar polymeric matrix on the efficiency of the immobilized phosphonium salts", pages 6606-6613. * Pages 6606-6613 * -- | 9 | CATEGORY OF CITED DOCUMENTS X: particularly relevant A: technological background O: non-written disclosure P: intermediate document T: theory or principle underlying the invention E: conflicting application D: document cited in the application L: citation for other reasons |
| | CHEMICAL .REVIEWS, vol. 77, no. 2, April 1977, V. AMARNATH et al. "Chemical synthesis of oligonucleotides" pages 183-217. * Pages 183-217 * -- ./. | 1 | |
| ✗ | The present search report has been drawn up for all claims | | &: member of the same patent family, corresponding document |

| Place of search | Date of completion of the search | Examiner |
|---|---|---|
| The Hague | 10-07-1981 | VERHULST |

EPO Form 1503.1 06.78